# EUROPEAN PATENT APPLICATION

(11) **EP 2 656 877 A1**
(43) Date of publication of application: **30.10.2013**
(21) Application number: 11851068.4
(22) Date of filing: 28.03.2011
(51) Int. Cl.: A61N 1/39, A61H 31/00

(54) **DEVICE FOR URGENT CARDIAC ASSISTANCE**

(30) Priority: 24.12.2010 ES 201031940
(71) Applicant: Merce Vives, Salvador, 46004 Valencia (ES)
(72) Inventor: Merce Vives, Salvador, 46004 Valencia (ES)
(74) Representative: Ungria López, Javier
(86) International application number: PCT/ES2011/070208
(87) International publication number: WO 2012/085306

(57) **Abstract**

The device (1) has a massager formed by a manual gripping body (2) that connects by means of a number of arms (4) to respective massaging end pieces (3), the device (1) including a system (5) for generating mechanical vibrations, which transmits mechanical vibrations to said massaging end pieces (3). Furthermore, the device comprises a system (6) for generating electrical discharges which can be applied to a patient's body by means of electrodes (7) located in said massaging end pieces (3).

## Description

### OBJECT OF THE INVENTION

The present invention, such as it is expressed in the statement of this specification, is related to a device for urgent cardiac assistance the essential purpose of which consists of enabling quick actions by personnel not specialised in emergency situations to anomalies of the circulatory system such as cardiac arrest, until the arrival of teams of specialised assistance. The invention enables the generation of a cardiac massage by means of an alternating and synchronizable electrical stimulation with enough energy to generate muscle contractions and cardiac massages of short duration.

### BACKGROUND OF THE INVENTION

Currently, in the presence of cardiac arrest the emergency procedures used are:
- Application of external massage by alternately pushing the patient's chest for achieving a cardiac massage.
- Application of artificial respiration, known as mouth-to-mouth.

These known procedures have disadvantages such as that the external massage and the artificial respiration, even when they are carried out by "lifeguards" or trained staff have a very low success rate.

On the other hand, to produce a synchronizable cardiac pulse and not compete with an eventual heart rate that starts autonomously by the heart subjected to the external stimulation, the frequency of the massager device must be synchronizable and variable, according to the pulse of the heart in rehabilitation.

The defibrillator as urgency device for cardiac assistance is known, while said device has drawbacks relating to that it only provides a very high quantity of energy in a very short time period, this is a high voltage electrical discharge, not allowing the possibility of assisting the heart during a time period in which its resuscitation is being attempted by means of the input of an alternate energy that provokes a cardiac massage, as well as determining the possible reaction of the same and the simultaneous synchronization with said spontaneous heart response for not hindering the stabilization of the same, but contributing to it. These conventional defibrillators have drawbacks in addition to that they should be used by a professional who clearly knows a series of initial guidelines, as well as having a clear understanding of the functioning of the apparatus they are handling.

Furthermore, some Industrial Property Registrations are known which are related to the machines and apparatus used in medicine for the heart, such as for example the patents with publication numbers ES-A3-0387307, ES-B1-2172419 and ES-T3-2097334, all of them related with heart defibrillation.

### DESCRIPTION OF THE INVENTION

To achieve the objectives and avoid the drawbacks referred to in previous paragraphs, the invention consists of a device for urgent cardiac assistance where the device has a massager formed by a manual gripping body that is connected by means of a number of arms with respective massaging end pieces, including in the device a system for generating mechanical vibrations which transmits mechanical vibrations to the aforementioned massaging end pieces.

Innovatively, according to the invention, the system of the same comprises a generator of electrical discharges which can be applied to a patient's body by means of electrodes located in the aforementioned massaging end pieces.

According to a preferred embodiment of the invention, the aforementioned system for generating electrical discharges is incorporated inside the aforementioned gripping body.

In other embodiments of the invention, the system for generating electrical discharges is incorporated in an external console independent from the massager and connectable to the same by means selected from: wired, wireless and any combination of both.

Furthermore, in the preferred embodiment of the invention, the mentioned arms are arranged in groups of three.

On the other hand, in the preferred embodiment of the invention the generator of electrical discharges has means of synchronization of the following frequencies: frequency of the system for generating vibrations, frequency of the system for generating electrical discharges, heart rate of the referred patient and any combination of the same.

With the structure that has been described, the device for urgent cardiac assistance of the invention has advantages related to that it can be used safely by any non-medical professional user, with a chance of success in their performance far superior to that of the current external massages applied on the chest and mouth-to-mouth respiration. Other advantages of the invention consist of the great ease of application and the low economic cost, both of the manufacture of the device, and of having one or more of said devices in optimal conditions for possible use.

Apart from the advantages listed above we would like to point out in this part of the specification that the aforementioned system for generating electrical discharges connected to the electrodes can be configured in a block including a controller circuit controlling an information display screen and connecting with an electrical energy generator which in turn controls several power transmission circuits by means of which are established respective connections to the aforementioned electrodes.

Next, to facilitate a better understanding of this specification and forming an integral part of the same, a single figure is accompanied in which with illustrative character and without limitation the object of the invention has been represented.

### BRIEF DESCRIPTION OF THE FIGURE

**Figure 1****.-** It shows a perspective view of a device for urgent cardiac assistance carried out according to the present invention, showing in a schematic way the functional blocks that it includes inside.
**Figure 2****.-** It shows the functional block diagram of the part corresponding to the electric massage or shock to the heart facilitated by the electronics of the device of the previous figure 1; in this figure, the electronics corresponding to the electromechanical or vibration massage are not shown.

### DESCRIPTION OF AN EXAMPLE OF EMBODIMENT OF THE INVENTION

Below, there is a description of an example of the invention alluding to the references of the figures.

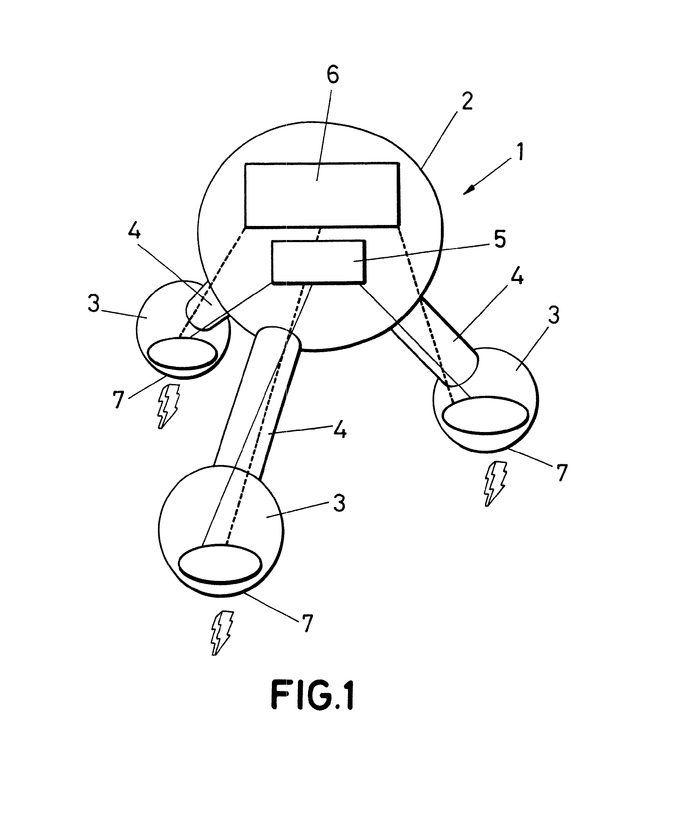

Thus, the device for urgent cardiac assistance of this example of the invention uses a conventional hand massager with an approximately spherical manual gripping body 2 from which three arms 4 angularly equidistant according to three orthogonal axes extend, finishing each of said arms 4 in respective massaging end pieces 3, also approximately spherical, but with a size considerably smaller than the gripping body 2, such as it has been plotted in figure 1.

Furthermore, the device 1 with its gripping body 2, its arms 4 and its massaging end pieces 3 is equipped with a system for generating mechanical vibrations 5, such as some of these conventional massagers currently are.

The main feature of the present example of the invention consists of incorporating in addition to said system of mechanical vibrations 5, and also within the gripping body 2 a system for generating electrical discharges 6 susceptible to various modes of operation and operability, given the possibilities of the state of the art in the electronics sector; said system for generating electrical discharges 6 being connected to three electrodes 7, each located in a different end massager 3, which offers a great range of possibilities of electrical discharges according to the variations of the electrical potentials of said electrodes 3.

However, for other embodiments, and being obvious to a person with average skill in the art to put it into practice, the system for the generation of electrical discharges can be arranged in an external console that is connected wired, wirelessly or with any combination of said connectivity means with the gripping body of the prototype of the invention.

On the other hand, although in this example the aforementioned arms 4 and electrodes 7 are arranged in groups of three, it is also clear for a person with average skill in the art that said number could be sized differently in other embodiments.

Another feature of the present example of the invention consists in that the referred system for generating electrical discharges 6 has means of synchronization of the following frequencies: frequency of the system for generating vibrations 5, frequency of the system for generating electrical discharges 6, heart rate of the referred patient and any combination of the same. For the cases in which said heart rate of the patient is used, the system for generating electrical discharges 6 includes a corresponding sensor that can perform its detection through the electrodes 7.

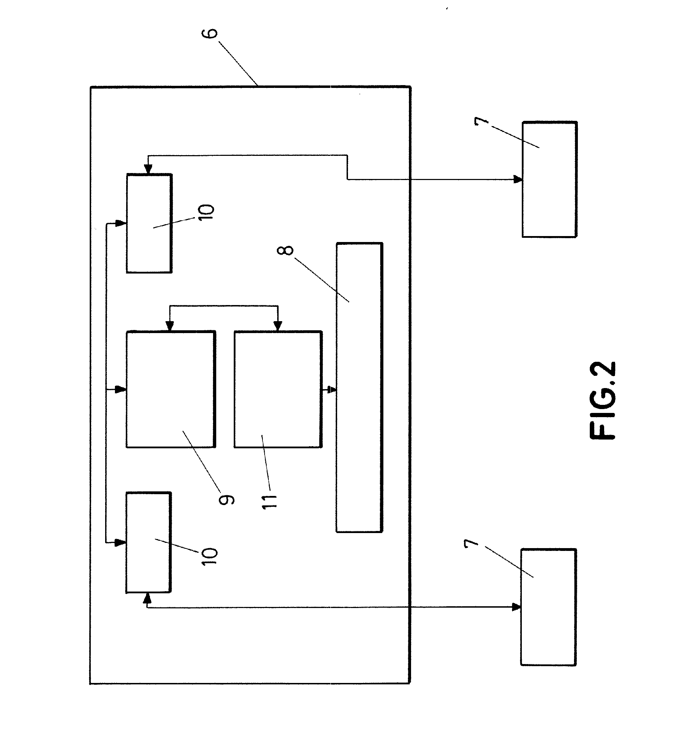

Furthermore, in the present example of the invention the system for generating electrical discharges 6 can be formed, such as it is shown in figure 2, by a controller circuit 11 which is in turn connected to an information display screen 8 and to an electrical energy generator 9; while the latter electrical energy generator 9 is joined to power transmission circuits 10 by means of which are established respective connections with the electrodes 7 mentioned above; in the aforementioned figure 2 these power transmission circuits 10 have been dimensioned in groups of two, although the dimensioning could be in another number.

The device of the invention shown in the present example provides the advantage of controlling the stimulation with regard to power and frequency of the stimulus, by means of a software, since it is possible, in an electronic manner similar to that existing for other medical applications, to control cardiac behaviour, so that the necessary frequency and power are delivered in the corresponding massage.

The device for urgent cardiac assistance of the present example has a perfect applicability in the modern society, since thousands of deaths are annually produced in Spain caused by strokes or cardiac attacks, of vascular nature and due to a late attendance of the patient. Furthermore, the device of the present example facilitates immediate medical assistance at home, at accident locations and in many other places, such as e.g. hospitals, without being necessary the participation of a practitioner.

The device of the present example has an additional complement to the classic defibrillators to cardiacally help when the heart has responded to the initial charge of the defibrillator. Furthermore, the device of electric massage of this example can work or not in conjunction with the part corresponding to the mechanical massage.

A box that allows operation with batteries and an AC mains adapter is also foreseen in the prototype of the present example.

## Claims

1. Device for urgent cardiac assistance, wherein the device (1) has a massager formed by a manual gripping body (2) that connects by means of a number of arms (4) to respective massaging end pieces (3), the device (1) including a system (5) for generating mechanical vibrations which transmits mechanical vibrations to said massaging end pieces (3); **characterized in that** it comprises a system (6) for generating electrical discharges which can be applied to a patient's body by means of electrodes (7) located in said massaging end pieces (3).

2. Device for urgent cardiac assistance, according to claim 1, **characterized in that** said system (6) for generating electrical discharges is incorporated inside the manual gripping body (2).

3. Device for urgent cardiac assistance, according to claim 1, **characterized in that** said system (6) for generating electrical discharges is incorporated in an external console independent from the massager and connectable to the same by means selected from: wired, wireless and any combination of both.

4. Device for urgent cardiac assistance, according to any one of the previous claims, **characterized in that** said arms (4) are arranged in groups of three.

5. Device for urgent cardiac assistance, according to any one of the previous claims, **characterized in that** the system (6) for generating electrical discharges has means of synchronization of the following frequencies: frequency of the system for generating vibrations (5), frequency of the system for generating electrical discharges (6), heart rate of a patient and any combination of the same.

6. Device for urgent cardiac assistance, according to any one of the previous claims, **characterized in that** the aforementioned system (6) for generating electrical discharges connected to the electrodes (7) is configured in a block including a controller circuit (11) controlling an information display screen (8) and connecting with an electrical energy generator (9) which in turn controls several power transmission circuits (10) by means of which are established respective connections to the electrodes (7).
